# EUROPEAN PATENT APPLICATION

(11) **EP 4 596 706 A1**
(43) Date of publication of application: **06.08.2025**
(21) Application number: 23872594.9
(22) Date of filing: 29.09.2023
(51) Int. Cl.: C12Q 1/04, C12N 5/10, C12N 5/12, C12P 21/08, G01N 33/53

(54) **SCREENING METHOD FOR CELL THAT PRODUCES OBJECTIVE SUBSTANCE, NUCLEIC ACID PRODUCTION METHOD, AND OBJECTIVE SUBSTANCE PRODUCTION METHOD**

(30) Priority: 30.09.2022 JP 2022158723
(71) Applicant: FUJIFILM Corporation, Tokyo 106-8620 (JP)
(72) Inventor: ENDO Koki, Tokyo 106-8620 (JP); KANDA Rikiya, Tokyo 106-8620 (JP); MACHIDA Yuki, Tokyo 106-8620 (JP)
(74) Representative: Hoffmann Eitle
(86) International application number: PCT/JP2023/035596
(87) International publication number: WO 2024/071374

(57) **Abstract**

An object of the present invention is to provide a screening method for a cell, which has excellent screening sensitivity and excellent screening efficiency, a manufacturing method for a nucleic acid, in which the nucleic acid is acquired from a cell obtained by the screening method, and a manufacturing method for an objective substance, in which an objective substance is manufactured using the nucleic acid. According to the present invention, provided is a screening method for a target cell which is a cell that produces an objective substance, the screening method including the following (1) to (3), (1) immobilizing a vesicle having a target substance on a membrane surface in a microwell, (2) introducing a candidate cell obtained from a cell population containing the target cell and a labeled substance which is a substance that binds to the objective substance into the microwell in which the vesicle has been immobilized, and (3) identifying the target cell by detection of a label of the objective substance bound to the target substance.

## Description

### Technical Field

The present invention relates to a screening method for a cell producing an objective substance, a manufacturing method for a nucleic acid, and a manufacturing method for an objective substance.

### Background Art

**In** recent years, a search for a substance (also referred to as a "specific binding substance") that specifically binds to a protein such as a cell membrane protein has been performed for various purposes. Examples of the specific binding substance include an antibody and a protein ligand.

Such a specific binding substance is used, for example, as a research reagent, a diagnostic agent, a therapeutic agent, or the like.

As a technique for searching for the specific binding substance, for example, a technique in which all or a part of a protein is immobilized in a well and binding to a substance which is a candidate for the specific binding substance is evaluated by enzyme-linked immunosorbent assay (ELISA) or the like has been performed.

**In** addition, for example, as a search method in consideration of the three-dimensional structure of the cell membrane protein, in the above-described ELISA and the like, evaluation of binding to a substance that is a candidate for a specific binding substance using cells in which instead of all or a part of the above-described proteins, the target cell membrane protein is expressed on the cell membrane.

On the other hand, the above-described specific binding substance such as a ligand or an antibody can be secreted into a culture supernatant by culturing a cell derived from a human or a non-human animal, or a recombinant cell to which a gene recombination technique is applied.

Here, in a case of searching for a cell that produces an unknown substance which specifically binds to a target protein, it is necessary to make a production cell population (for example, an antibody-producing hybridoma library) containing a total of several thousands to several hundreds of thousands of cells, as a production cell population containing cells that produce a substance which is a candidate for a specific binding substance, investigation target.

As such a search method, Patent Document 1 describes a method of isolating a single antibody-producing particle having specificity for a selected antigen, the method including: (1) preparing vesicles, preferably exosomes, displaying the selected antigen and a marker; (2) bringing the vesicles in the step 1 into contact with an antibody repertoire; and (3) identifying and isolating a single antibody-producing particle that has reacted with the vesicles.

Patent Document 2 describes a method for selecting cells, in which a target cell that produces an objective substance which specifically binds to a desired cell membrane protein is selected from a population of second cells, the method including: a) a step of providing a substrate having a plurality of microwells; b) a step of attaching first cells that express the cell membrane protein on a cell surface to each of the microwells; c) a step of introducing one or two second cells isolated from the population into each of the microwells to allow the first cells and the second cells to coexist in the microwells, subsequent to the step b); d) a step of identifying a microwell containing the first cells to which the objective substance is bound, subsequent to the step c); and e) a step of collecting the second cells as the target cell from the microwell identified in the step d).

### Prior Art Documents

### Patent Documents

Patent Document 1: JP2006-518212A
Patent Document 2: WO2020/171020A

### SUMMARY OF THE INVENTION

### Object to be solved by the invention

As described above, in a case of searching for a cell that produces an unknown substance which specifically binds to a target protein, it is necessary to screen a target cell from a production cell population containing several thousands to several hundreds of thousands of cells, and improvement of screening sensitivity (high positive rate and few false negatives) and improvement of screening efficiency (that is, more efficient selection of cells) are required.

An object of the present invention is to provide a screening method for a cell, which has excellent screening sensitivity and excellent screening efficiency, a manufacturing method for a nucleic acid, in which the nucleic acid is acquired from a cell obtained by the screening method, and a manufacturing method for an objective substance, in which an objective substance is manufactured using the nucleic acid.

### Means for solving the object

Representative aspects of the present invention are shown below. However, the present invention is not limited thereto.
<1> A screening method for a target cell which is a cell that produces an objective substance, the screening method comprising the following (1) to (3):
   (1) immobilizing a vesicle having a target substance on a membrane surface in a microwell;
   (2) introducing a candidate cell obtained from a cell population containing the target cell and a labeled substance which is a substance that binds to the objective substance into the microwell in which the vesicle has been immobilized; and
   (3) identifying the target cell by detection of a label of the objective substance bound to the target substance.
<2> The screening method according to <1>, in which the target substance is a membrane protein.
<3> The screening method according to <1> or <2>, in which the objective substance is an antibody.
<4> The screening method according to any one of <1> to <3>, in which the target cell is a B cell derived from a spleen cell or lymphoid tissue.
<5> The screening method according to any one of <1> to <3>, in which the target cell is a CHO cell or a hybridoma.
<6> The screening method according to any one of <1> to <5>, in which in the (1), the vesicle is immobilized in the microwell using at least one selected from the group consisting of a Tim family protein, annexin V, an anti-CD9 antibody, an anti-CD63 antibody, and an anti-CD81 antibody.
<7> The screening method according to <6>, in which in the (1), the vesicle is immobilized in the microwell using the Tim family protein.
<8> The screening method according to any one of <1> to <7>, in which in the (2), the candidate cell to be introduced into the microwell is one cell that has been isolated.
<9> The screening method according to any one of <1> to <8>, in which the vesicle is an extracellular vesicle.
<10> The screening method according to any one of <1> to <9>, in which the vesicle has a diameter of 30 to 1,000 nm.
<11> The screening method according to any one of <1> to <10>, in which the detection in the (3) is fluorescence detection.
<12> The screening method according to <11>, in which the identifying of the target cell by the fluorescence detection is performed by excitation at one wavelength and fluorescence measurement at one wavelength.
<13> The screening method according to <11> or <12>, in which in the (3), the target cell is identified by detecting a well in which fluorescence is confirmed at a position of a side wall of the microwell.
<14> The screening method according to any one of <1> to <13>, further comprising the following (4):
   (4) collecting the target cell identified in the (3).
<15> A manufacturing method for a nucleic acid comprising:
   acquiring a nucleic acid encoding an objective substance from the target cell obtained by the screening method according to any one of <1> to <14>.
<16> A manufacturing method for an objective substance, comprising:
   manufacturing an objective substance using the nucleic acid obtained by the manufacturing method for a nucleic acid according to <15>.

### Effect of the Invention

According to the present invention, a screening method for a cell, which has excellent screening sensitivity and excellent screening efficiency, a manufacturing method for a nucleic acid, in which the nucleic acid is acquired from a cell obtained by the screening method, and a manufacturing method for an objective substance, in which an objective substance is manufactured using the nucleic acid, are provided.

### BRIEF DESCRIPTION OF THE DRAWINGS

Fig. 1 is graphs each showing an analysis result of a particle diameter distribution in Example 1.
Fig. 2 is a graph showing values of fluorescence intensity in Example 2.
Fig. 3 is diagrams each showing an example of imaging results in Example 3.
Fig. 4 is a diagram showing an example of imaging results of a negative cell in Example 3.
Fig. 5 is a diagram showing an example of imaging results of a positive cell observed by Cell-ELISA in Example 4.
Fig. 6 is a diagram showing an example of imaging results of a negative cell observed by Cell-ELISA in Example 4.
Fig. 7 is a graph showing the absorbance measurement results in Example 5.
Fig. 8 is graphs each showing the absorbance measurement results in Example 6.
Fig. 9 is a graph showing values of S/B ratios in Example 6.
Fig. 10 is a graph showing the measurement results of antibody activity in Example 7.

### EMBODIMENTS FOR CARRYING OUT THE INVENTION

### (Screening method for cell)

The screening method according to the embodiment of the present invention is a screening method for a target cell which is a cell that produces an objective substance, and includes the following (1) to (3):
(1) immobilizing a vesicle having a target substance on a membrane surface in a microwell,
(2) introducing a candidate cell obtained from a cell population containing the target cell and a labeled substance which is a substance that binds to the objective substance into the microwell in which the vesicle has been immobilized, and
(3) identifying the target cell by detection of a label of the objective substance bound to the target substance.

Hereinafter, the vesicle having the target substance to which the objective substance is bound on the membrane surface is also simply referred to as "EV".

According to the present invention, a screening method having excellent screening sensitivity and excellent screening efficiency is provided.

Here, for example, JP2006-518212A describes that a vesicle in which the selected antigen and marker are displayed is used. There is a limit to the expression level of the antigen that can be expressed in the vesicle and the expression level of the marker, and it is difficult to identify a cell that produces an objective substance with high sensitivity.

Furthermore, the method described in JP2006-518212A is a method using flow cytometry. Here, in the method using flow cytometry, there are also issues that secreted type antibody-producing cells and the like cannot be acquired, a determination criterion (threshold value) of whether or not the cell is positive is ambiguous, it is difficult to reliably collect one cell, damage to cells is large, and false positives due to non-specific fluorescence occur.

In the method described in WO2020/171020A, it is necessary to put two cells, a first cell that expresses a cell membrane protein such as a Chinese Hamster Ovary (CHO) cell and a second cell that produces an objective substance such as a B cell, in one microwell. Therefore, it is necessary to increase the size of the microwell itself, and the number of wells present on the substrate is reduced.

In addition, since the analysis can be performed only in a well containing both the cell expressing the cell membrane protein and the cell producing the objective substance, the number of wells that can be analyzed is reduced.

As described above, in the method described in WO2020/171020A, there is a limit to the number of wells that can be used for the analysis, and it is difficult to identify a cell that produces an objective substance with high efficiency.

On the other hand, in the screening method according to the present invention, various known labels having excellent detection ability can be used as the label to be detected.

In addition, as compared with a method using flow cytometry, the screening method according to the present invention has advantages that a secreted type antibody-producing cell or the like can be acquired, the determination criteria for whether or not the cell is positive is clear since it is possible to determine whether or not the cell is positive based on the detected shape, it is easy to collect one cell, the damage to the cell is small, and false positive due to non-specific fluorescence is less likely to occur.

In addition, in the screening method according to the present invention, since only the candidate cells need to be introduced into the microwell in which the EV is immobilized, the number of wells present on the substrate can be increased by reducing the size of the microwell.

Furthermore, in the screening method according to the present invention, since the EV can be easily immobilized in the microwell, any microwell in which the candidate cell is introduced can be used for the analysis. That is, as compared with the method described in WO2020/171020A in which both the first cell and the second cell need to be placed in one microwell, the number of wells that can be analyzed is increased.

As described above, it can be said that the screening method according to the embodiment of the present invention is excellent in screening sensitivity and screening efficiency as compared with the related art.

Hereinafter, the details of the screening method according to the embodiment of the present invention will be described. In the present invention, mol/l is also described as M. Similarly, "mmol/l" and "µmol/l" are also described as "mM" and "µM".

In addition, in the present invention, a combination of preferable aspects is a more preferable aspect.

### < Cell producing objective substance (target cell) >

The target cell is a cell that produces an objective substance.

### [Objective substance]

Examples of the objective substance include a polypeptide, a cyclic peptide, a protein, and the like, which selectively bind to a target substance.

More specific examples thereof include peptide hormones, cytokines, antibodies, artificial polypeptides, artificial cyclic peptides, and the like.

Among these, the objective substance is preferably an antibody or a ligand, and more preferably an antibody.

In the present invention, the term "antibody" can be replaced with "immunoglobulin". The antibody in the present invention also includes functional fragments of the antibody.

Here, the "functional fragment of an antibody" refers to a partial fragment of an antibody (that is, an immunoglobulin), which retains at least one action on an antigen.

Examples of the partial fragment include F(ab')₂, Fab, Fv, a disulfide-bonded Fv, a single-chain antibody (scFv, VH-VL), VH, VHH, polymers thereof, a fusion thereof with a heavy chain CH3 region, and the like.

The antibody may be a multispecific antibody. Examples of the multispecific antibody include a diabody, which is a type of bispecific antibody (for example, WO1993/011161A), and the like.

The class (isotype) of the antibody in the present invention is not particularly limited. For example, any classes such as IgG, IgM, IgA, IgD, or IgE may be used.

Furthermore, the subclass of the antibody is also not particularly limited. For example, IgG may be any subclasses such as IgG1, IgG2, IgG3, or IgG4.

Furthermore, the antibody may be any of a fully human antibody, a humanized antibody, or a chimeric antibody.

### [Target cell and cell population containing target cell]

The target cell is not particularly limited as long as it is a cell expected to produce a desired objective substance.

The cell population containing the target cell may be a cell population that may contain the target cell, and the target cell may not actually be contained.

### -Antibody-producing cell-

In a case where the objective substance is an antibody, the target cell is an antibody-producing cell.

Examples of the antibody-producing cell include a B cell or plasma cell derived from a spleen cell, lymphoid tissue, or blood, a hybridoma, a CHO cell, and the like.

As the B cell or plasma cell derived from splenic cells, lymphoid tissues, or blood, those collected from healthy humans or animals, humans or animals suffering from cancer, humans or animals suffering from known or unknown infectious diseases, humans or animals suffering from autoimmune diseases, humans or animals vaccinated with a vaccine, and the like can be used. For example, this collected cell population can be used as the cell population containing the above-described target cell.

Among these, an aspect in which the target cell is a B cell derived from a spleen cell or lymphoid tissue is one of the preferred aspects of the present invention.

To more efficiently identify the target cell for the target substance, cell concentration may be performed. For example, activated B cells or plasma cells, which are obtained from bone marrow, spleen, lymphoid tissue, or blood cells derived from a non-human animal immunized with a target substance can be concentrated and used as a cell population containing the above-described target cells.

In addition, for example, B cells (particularly, activated B cells) or plasma cells obtained from human lymphoid tissues or blood-derived cells can be concentrated and used as a cell population containing the target cells.

The concentration of the activated B cells or the plasma cells can be performed, for example, using a CD antigen on the cell surface as an indicator. For example, the concentration can be performed using antibody magnetic beads for a specific CD antigen. For example, the activated B cells or the plasma cells may be concentrated by a negative selection method using one or two or more types of antibody magnetic beads for CD2, CD3, CD4, CD8, CD11b, CD11c, CD14, CD15, CD16, CD34, CD40, CD43, CD45R, CD49b, CD56, CD61, CD79a, CD90.2, CD138, CD235a, and the like, or the activated B cells or the plasma cells may be concentrated by a positive selection method using one or two or more types of antibody magnetic beads for CD19, CD20, CD25, CD27, CD38, CD78, CD138, CD319, and the like.

The degree of concentration is not particularly limited, but for example, the B cells or the plasma cells can be concentrated by 50 times or more from a lymphocyte cell population derived from lymphoid tissue of about 10,000,000 cells.

There are various methods for immunizing a non-human animal with a target substance, as shown in Hutchings CJ, Koglin M, Olson WC, Marshall FH, "Opportunities for therapeutic antibodies directed at G-protein-coupled receptors", Nat. Rev. Drug Discov. 16 (9), 2017.

For example, examples thereof include a method of synthesizing a partial peptide or a partial protein, which is exposed on the cell surface and using the partial peptide or the partial protein as an antigen for immunization. In addition, examples thereof include a method of solubilizing a target substance from cells with a surfactant, purifying the solubilized target substance, and using the purified target substance as an antigen for immunization. In addition, examples thereof include a method of directly immunizing a cell itself in which a target substance is highly expressed. In addition, examples thereof include a method of performing immunization using a target substance presented on an artificial bilayer or virus-like nanoparticles as an antigen. In addition, examples thereof include a method of immunizing a vesicle, in which the target substance used in (1) according to the embodiment of the present invention is used on a membrane surface, as an antigen. Furthermore, examples thereof include a method of immunizing a protein expression vector with a vector in which a cDNA sequence encoding a target substance is inserted (DNA immunization).

Among these, DNA immunization is preferable since it has a high probability of acquiring an antibody having higher specificity and higher affinity.

An aspect in which the target cell is a CHO cell or a hybridoma is also one of the preferred aspects of the present invention.

The CHO cell is preferably a recombinant cell into which an antibody gene has been introduced.

The recombinant cell is produced, for example, by the following procedure.

A cDNA library is produced from the B cell or plasma cell derived from lymphoid tissues or blood cells of immunized animals. A gene of an antibody or an antibody fragment is selectively amplified from the cDNA library. The amplified gene is modified such that various forms of antibody molecules such as a complete antibody, a functional antibody fragment, a single-chain antibody, or a multispecific antibody can be expressed, thereby producing an antibody gene library. This gene library is incorporated into a vector such as pcDNA, pEF/FRT/V5-DEST, or Mammalian PowerExpress System. Then, this vector is introduced into CHO cells to obtain recombinant cells.

A cell population containing the recombinant cell can be used as the cell population containing the target cell described above.

Furthermore, in the recombinant cell, a cell population containing a recombinant cell that has survived by using a drug resistance gene carried by the vector and that constantly expresses a gene may be used as the cell population containing the above-described target cell.

**In** addition, in the present invention, the target cell may be a recombinant cell obtained by the same method using cells such as a COS cell, a HEK293T cell, and an NS0 cell instead of the CHO cell in the above-described method.

The target cell may be a hybridoma.

For example, an immune cell is collected from a non-human animal immunized with a target substance, and the immune cell is fused with myeloma cells to obtain a cell population containing a hybridoma. A cell population containing this hybridoma can be used as a cell population containing the above-described target cell.

For cell fusion, selection of hybridoma, and cloning, known methods can be used. For example, cell fusion can be performed by a method using polyethylene glycol or a method of applying a voltage to a mixed solution of an immune cell and a myeloma. **In** addition, the selection of the hybridoma can be performed by culturing using a HAT selective culture medium. This cell population after the selection can also be used as the cell population containing the above-described target cell.

The target cell may be a cell that has been immortalized by a method other than the hybridoma method. For example, in a case of a B cell derived from human lymphoid tissue or blood, the B cell may be a cell immortalized by Epstein-Barr virus infection. A cell population containing these cells can also be used as the cell population containing the above-described target cell.

### -Other cells-

**In** a case where the target cell is a cell other than the antibody-producing cell, examples of the target cell include various cells derived from non-human or human tissues, such as blood cells, nerve cells, vascular endothelial cells, vascular smooth muscle cells, immune cells, adipocytes, skeletal muscle cells, lymphocyte cells, skin cells, and the like.

For example, as a cell population containing the above-described target cell, a cell population obtained by separating a tissue of a non-human animal, isolating the tissue by a treatment with a proteolytic enzyme or the like, and then filtering the tissue through a mesh of 30 to 100 µm can be used.

For example, a cell population isolated from human blood or a surgically excised organ can also be used as the cell population containing the above-described target cell.

Furthermore, the target cell may be a tumor cell. For example, a cell population isolated from a surgically excised organ can also be used as the cell population containing the above-described target cell.

In addition, the cell population containing tumor cells can be obtained from ATCC or a cell sales company.

The target cell may be a recombinant cell different from the antibody-producing cell.

For example, a cDNA library containing a gene encoding an objective substance is incorporated into an expression vector such as pcDNA, pEF/FRT/V5-DEST, or Mammalian PowerExpress System. Then, this vector is introduced into cells such as CHO cells, COS cells, HEK293 cells, and NS0 cells to obtain a cell population containing recombinant cells. This cell population can also be used as the cell population containing the above-described target cell.

Furthermore, in the recombinant cell, a cell population containing a recombinant cell that has survived by using a drug resistance gene carried by the vector and that constantly expresses a gene can be used as the cell population containing the above-described target cell.

In addition, a cell population containing CHO cells, HEK293 cells, NIH3T3 cells, or the like infected with a virus vector derived from an adenovirus, a lentivirus, or the like, in which a cDNA library is incorporated can also be used as the cell population including the above-described target cell.

It is preferable that a gene encoding one type of objective substance is introduced into the recombinant cell.

The animal species from which the target cell is derived is not particularly limited, but a mammalian cell or a bird cell is preferably used. Examples of the mammal include a mouse, a rat, a guinea pig, a rabbit, a monkey, a cow, a horse, a dog, a cat, a goat, a sheep, a pig, a camel, an alpaca, and the like. In addition, examples of the birds include a chicken, a duck, and a turkey.

### <(1)>

The screening method according to the embodiment of the present invention includes (1) immobilizing a vesicle having a target substance on a membrane surface in a microwell.

### [Vesicle having target substance on membrane surface]

### -Target substance-

The target substance in the vesicle having the target substance on the membrane surface is preferably a membrane protein. The membrane protein is not particularly limited, such as a cell membrane protein, a lysosomal membrane protein, a ribosomal nuclear protein, or a mitochondrial membrane protein, but is preferably a cell membrane protein.

In addition, the target substance may be any protein other than a membrane protein such as an enzyme, or may be a compound other than a polypeptide, such as a glycolipid, polysaccharides, a drug, and an organic chemical substance.

In addition, the target substance is preferably an antigen or a receptor for an objective substance (an antibody or a ligand).

The cell membrane protein is not particularly limited, and all cell membrane proteins represented by a multiple pass transmembrane protein can be targeted.

Examples thereof include a G protein-coupled receptor (GPCR), an ion channel, a transporter, a CD antigen, a cell adhesion molecule, a cancer antigen, a virus antigen, and the like.

In addition, the animal species from which the cell membrane protein is derived is not particularly limited to.

Furthermore, in the present invention, a cell membrane protein having circumstances such as a case where a purification method has not been established, a case where large-scale purification is difficult, or a case where isolation and purification in a state of maintaining a structure naturally present is difficult, the protein of which a part is exposed from a lipid bilayer to the extracellular space, can be a target.

### -Vesicle-

The vesicle is not particularly limited as long as it has a target substance on the surface, but is preferably a vesicle composed of a lipid bilayer.

In addition, from the viewpoint of immobilizing the vesicle in the microwell, the vesicle preferably has at least one selected from the group consisting of tetraspanin such as CD9, CD63, or CD81 and phosphatidylserine on the membrane surface, and more preferably has phosphatidylserine on the membrane surface.

The diameter of the vesicle is preferably 30 to 1,000 nm, more preferably 50 to 800 nm, still more preferably 50 to 500 nm, and even still more preferably 50 to 200 nm.

In the present invention, the diameter of the vesicle can be measured, for example, by a nanoparticle tracking analysis method (NTA method) using a nanoparticle analysis system (Nanosight). The diameter of the vesicle refers to a number average particle size.

The vesicle is not particularly limited, but is preferably an extracellular vesicle.

The extracellular vesicle is a cell-derived small membrane vesicle composed of a lipid bilayer.

Examples of the extracellular vesicle include various extracellular vesicles classified according to their origin and size of small membrane vesicles, as described in Nature Reviews Immunology 9, 581-593 (August 2009), "Obesity Research" Vol. 13 No. 2 2007, topics by Naoto Aoki et al., or the like. Specific examples thereof include exosomes, microvesicles, ectosomes, membrane particles, exosome-like vesicles, apoptotic bodies, adiposomes, and the like, and exosomes, microvesicles, or apoptotic bodies are preferable and exosomes are more preferable.

The exosome is a cell-derived small membrane vesicle composed of a lipid bilayer, and for example, each exosome has a diameter of 30 nm to 200 nm, preferably has a diameter of 30 nm to 150 nm, and more preferably has a diameter of 30 nm to 100 nm. The exosomes are considered to be derived from late endosomes.

The microvesicle is a cell-derived small membrane vesicle composed of a lipid bilayer, and for example, each microvesicle has a diameter of 100 nm to 1000 nm, preferably has a diameter of 100 nm to 800 nm, and more preferably has a diameter of 100 nm to 500 nm. The microvesicles are considered to be derived from cell membranes.

The apoptotic body is a cell-derived small membrane vesicle composed of a lipid bilayer, and for example, each apoptotic body has a diameter of 50 nm to 1,000 nm, preferably has a diameter of 50 nm to 800 nm, and more preferably has a diameter of 50 nm to 500 nm.

The vesicles can be obtained, for example, by a method of collecting vesicles from a culture supernatant after overexpressing a target substance in a vesicle-producing cell by a known method, a method of collecting vesicles from a body fluid of a human or an animal, or the like.

The vesicle-producing cell is not particularly limited, but examples thereof include cell known to have a high efficiency of gene transfer, such as 293T cells and CHO cells, and the like. In addition, known cells such as other antibody-producing cells can be used without particular limitation.

As a method of overexpressing a target substance in a vesicle-producing cell, a known method can be used without particular limitation.

For example, a gene construct (or vector) encoding a target substance may be introduced into the vesicle-producing cell by a known transfection method or the like. Examples of the introduction method include a conventional method such as a naked DNA expression technique, cationic lipid-mediated transfection, polymer-mediated transfection, peptide-mediated transfection, virus-mediated transfection, a physical or chemical reagent or treatment, and electroporation.

The method of collecting the vesicles is not particularly limited, and a known method may be used. Examples thereof include a method of subjecting a sample containing vesicles such as a culture supernatant or a body fluid to ultracentrifugation to obtain extracellular membrane vesicles as a precipitated fraction, a method of collecting vesicles by ultrafiltration using a membrane with a pore size selected according to the diameter of the vesicles to be acquired, a method of acquiring vesicles by affinity between a surface antigen protein and an antibody using an antibody against the surface antigen protein of the vesicles (for example, an anti-CD63 antibody), a method using a Tim family protein bound to a carrier (for example, a magnetic bead), as described in JP2021-012200A and the like, and the like.

In addition, in the method of collecting the vesicles, a commercially available kit such as MagCapture (registered trademark) Exosome Isolation Kit PS Ver. 2 (manufactured by FUJIFILM Wako Pure Chemical Corporation, 290-84103) may be used.

### -Microwell-

The microwell refers to a micro-sized well (dent, recess) in which 1 to 3 mammalian cells or avian cells can be contained. The microwell is a bottomed small hole, and for example, the inner diameter of the opening portion thereof is preferably about 10 µm to 50 µm, more preferably 10 to 30 µm, and still more preferably 15 to 25 µm. The depth of the microwell is not particularly limited, but is preferably about the same as the inner diameter of the opening portion.

In addition, the diameter (inner diameter) of the opening portion of the microwell can be appropriately determined in consideration of the type of the cell (that is, the candidate cell) to be stored in the microwell.

The shape of the microwell is preferably a cylindrical shape. In addition, the shape of the microwell may be a polygonal cylindrical shape such as a square cylinder or a hexagonal cylinder, or a shape in which the cross-sectional area decreases from the opening portion to the bottom surface, of an inverted cone cylinder type or an inverted conical cylinder type. In addition, a shape in which two or more of these shapes are combined and connected may be used.

In a case where the microwell has a shape other than a cylindrical shape or an inverted conical shape, the inner diameter of the opening portion can be calculated as an equivalent circle diameter from the area of the opening portion. The equivalent circle diameter refers to a diameter of a circle having an area equal to the area of the figure.

It is preferable that a plurality of microwells are formed on the substrate.

The number (density) of the microwells per unit area on the substrate is not particularly limited, but for example, is preferably in a range of 20,000 to 200,000 per 1 cm² and more preferably in a range of 25,000 to 75,000 per 1 cm².

This density can be increased by using a microwell having a small inner diameter, and the screening efficiency can be increased.

The material of the substrate is not particularly limited, and for example, in a case where the detection in (3) described later is performed by fluorescence detection, a transparent material having low autofluorescence is preferable.

As the substrate on which the microwells are formed, a commercially available product can also be used. For example, a substrate (microwell chamber) having a plurality of microwells having an inner diameter of 10 µm, 20 µm, or 30 µm is commercially available from AS ONE Corporation.

### -Method of immobilizing vesicles in microwell-

The method of immobilizing the vesicles in the microwell is not particularly limited, but it is preferable to immobilize the vesicles in the microwell using a substance that binds to the vesicles.

The substance that binds to the vesicle is only required to be appropriately determined in consideration of the type of the vesicle, the type of the protein expressed on the surface of the vesicle, and the like, and examples thereof include a compound that binds to phosphatidylserine, such as a Tim family protein and annexin V, an antibody that binds to tetraspanin present on the surface of the vesicle, such as an anti-CD9 antibody, an anti-CD63 antibody, and an anti-CD81 antibody, and the like.

Among these, in (1), it is preferable to immobilize the vesicles in the microwells using any one or more selected from the group consisting of the Tim family protein, the annexin V, the anti-CD9 antibody, the anti-CD63 antibody, and the anti-CD81 antibody, and from the viewpoint of screening sensitivity, it is more preferable to immobilize the vesicles in the microwells using the Tim family protein (for example, Tim 4).

Specifically, for example, the vesicles can be immobilized on the microwell by coating the microwell with a substance that binds to the vesicles described above and then putting the vesicles in the microwell to react.

The coating can be performed, for example, by bringing a solution or suspension containing a substance that binds to the above-described vesicle into contact with the microwell. The time of contact, temperature conditions, and the like are not particularly limited, and examples of the method include a method of bringing the solution or suspension into contact with a microwell at 4°C for about one night.

The concentration of the substance that binds to the above-described vesicle in the solution or suspension is not particularly limited, and may be determined in consideration of the type of the substance, the detection sensitivity, the difficulty of occurrence of false positives and false negatives, and the like. For example, the concentration is preferably 1 to 100 µg/mL and more preferably 10 to 50 µg/mL.

In a case where the vesicles are reacted by being placed in the microwells, it is preferable that a suspension containing the vesicles is brought into contact with the microwells which have been subjected to the coating.

The content of the vesicles in the suspension is not particularly limited, and may be determined in consideration of the type of the substance, the detection sensitivity, the difficulty of occurrence of false positives and false negatives, and the like. The content of the vesicles is preferably 1 × 10⁹ to 1 × 10¹³ particles/mL, and more preferably 1 × 10¹⁰ to 1 × 10¹² particles/mL.

The time and conditions of the reaction are not particularly limited, and may be determined in consideration of the type and amount of the substance that binds to the above-described coated vesicle, the type and concentration of the vesicle, and the like, but for example, the reaction can be performed at room temperature for 2 hours.

Unless otherwise specified, the room temperature in the present specification refers to 23°C.

In addition, it is preferable that the coated and immobilized vesicles are present not only on the bottom surface of the microwell but also on the side surface (side wall) of the microwell. According to such an aspect, in a case of performing fluorescence detection in (3), it is possible to detect a well in which fluorescence is confirmed at the position of the side wall of the microwell.

Here, for the purpose of suppressing the non-specific reaction of the substance that binds to the objective substance in (2) described later, blocking may be further performed in (1).

For example, in the above-described aspect in which the microwell is coated with a substance that binds to a vesicle and then the vesicle is placed in the microwell to be reacted, blocking can be performed after the coating and before the reaction of the vesicle.

The blocking is not particularly limited, and can be performed using a known blocking agent. For example, in a case where the substance that binds to the objective substance in (2) described later is an antibody, skim milk or the like can be used.

The blocking time, the temperature during blocking, and the like may be according to a conventional method in ELISA.

### <(2)>

The screening method according to the embodiment of the present invention includes (2) introducing candidate cells obtained from a cell population containing the target cell and a substance that binds to the objective substance and is labeled into a microwell in which the vesicle is immobilized.

The candidate cell is a cell obtained from a cell population containing the target cell, and may be a cell corresponding to the target cell (that is, a cell producing the objective substance) or a cell not corresponding to the target cell (that is, a cell not producing the objective substance).

The number of candidate cells to be introduced into the microwell may be selected depending on the screening purpose or the like, but from the viewpoint of easily specifying the target cell as one cell, it is preferable that the candidate cells to be introduced into the microwell are one isolated cell.

The details of the cell population containing the target cell are as described above.

Examples of the labeled substance that is a substance that binds to the objective substance include an antibody, a receptor protein, and the like, and an antibody is preferable.

**In** addition, in (2), to indirectly detect the label in the labeled substance that binds to the objective substance, a compound that reacts with the label may be further reacted.

Specifically, an antibody that binds to the objective substance and has a label, and a compound that reacts with the label can be used.

Specific examples of such an aspect include the use of an antibody that binds to the objective substance and is labeled with biotin, and fluorescently labeled streptavidin, and the like.

The label in the antibody that binds to the objective substance and has a label is not particularly limited, and a known label can be used. For example, a label with a fluorescent substance, biotin, or the like can be used, and a label with a fluorescent substance or biotin is preferable.

**In** a case where the label is a label with a fluorescent substance, the direct fluorescence detection of the label (fluorescent substance) can be performed in (3) described later.

**In** a case where the label in the antibody that binds to the objective substance and has a label is a label with biotin, the indirect fluorescence detection of the label (biotin) can be performed in (3) described later by further reacting avidin, streptavidin, or the like, which is labeled with fluorescence.

The fluorescent substance in these aspects is not particularly limited, and examples thereof include Alexa Fluor (registered trademark), Aqua, Texas Red (registered trademark), fluorescein and a derivative thereof, rhodamine and a derivative thereof, Cascade Blue (registered trademark), phicoerythrin, DyLight (registered trademark), and the like.

### <(3)>

The screening method according to the embodiment of the present invention includes (3) identifying the target cell by detection of a label of the objective substance bound to the target substance.

**In** (3), the label of the composite body of the vesicle having the target substance on the membrane surface, the objective substance produced from the target cell, and the labeled substance that binds to the target substance is detected. As described above, the label may be directly detected by detecting the label itself, or may be indirectly detected by detecting a compound that is bound to the label.

The detection method in (3) may be selected depending on the type of the label, but is preferably fluorescence detection.

The excitation wavelength and the fluorescence wavelength in the fluorescence detection are not particularly limited, and may be selected depending on the type of fluorescence labeling.

One of the preferred aspects of the present invention is also to perform the identification of the target cell by the fluorescence detection by excitation at one wavelength and fluorescence measurement at one wavelength.

The identification of the target cell by the fluorescence detection by the excitation at one wavelength and the fluorescence measurement at one wavelength means that the data used for the identification of the target cell is data obtained by the excitation with the excitation light including one wavelength range and the fluorescence measurement in one wavelength range.

For example, in the method described in WO2020/171020A, the irradiation with excitation light for the fluorescence detection of the first cell and the measurement of fluorescence, and the irradiation with excitation light for the fluorescence detection of the second cell and the measurement of fluorescence are performed. That is, excitation at two types of wavelengths and fluorescence measurement at two types of wavelengths are performed.

However, in the screening method according to the embodiment of the present invention, only the composite body of the vesicle, the objective substance, and substance that binds to the objective substance is only required to be detected, and thus the target cell can be identified by excitation at one wavelength and measurement of fluorescence at one wavelength.

As described above, in the screening method according to the present invention, the number of times of irradiation with excitation light and fluorescence detection can be reduced as compared with the method described in WO2020/171020A, and thus the efficiency of the screening method can be improved.

**In** addition, since the number of times of irradiation with excitation light and fluorescence detection can be reduced, the measurement can be performed in a short time, and the damage to the target cell in the measurement can be reduced. That is, in a case where the target cell is collected in (4) described later, a target cell having a low degree of damage may be collected.

**In** (3), it is preferable to identify the target cell by detecting a well in which fluorescence is confirmed at a position of a side wall of the microwell.

Here, usually, the above-described coating and immobilized vesicles are also present on the side wall of the microwell.

Therefore, it is considered that a composite body of the vesicle, the objective substance, and the labeled substance that binds to the target substance, is also formed on the side wall of the microwell.

Furthermore, at the position of the side wall of the microwell, a plurality of the composite bodies are present in a stacked manner in the height direction. Therefore, in a case of being observed from the upper surface direction or the bottom surface direction of the microwell, it is considered that the fluorescence intensity at the position of the side wall is high as compared with the bottom portion of the microwell.

**In** addition, since there is at least a region where cells are not present or candidate cells are not present at the position of the side wall of the microwell, in a case where fluorescence at the position of the side wall is observed, it is possible to reduce the influence of detection by fluorescence derived from the candidate cells (for example, autofluorescence of negative cells, fluorescence observed by non-specific binding of a substance that binds to an objective substance, and the like).

**In** this way, by detecting fluorescence at the position of the side wall of the microwell, the fluorescence intensity is increased and the influence of the candidate cell can be reduced. Therefore, the detection sensitivity is increased, it is easy to determine whether the cell is positive or negative, and thus false positive and false negative are also unlikely to occur. That is, the sensitivity of the screening is excellent.

**In** such an aspect, it is also preferable that the fluorescence detection in (3) is the detection of fluorescence having the same shape as the shape of the orthography viewed from the observation direction of the side wall of the microwell.

For example, in a case where the microwell has a cylindrical shape, it is also preferable that the fluorescence detection in (3) is the detection of a ring-shaped fluorescence at the position of the side wall.

### <(4)>

It is also preferable that the screening method according to the embodiment of the present invention further includes the following (4).
(4) Collecting the target cell identified in the (3).

The target cells can be collected from the microwell, for example, using a micromanipulator. For example, a capillary having a diameter of several µm to 50 µm can be inserted into a microwell (positive microwell) in which the presence of the target cell is identified, and the target cell can be suctioned and collected while being alive.

The operation by the micromanipulator may be an automatic operation or a manual operation. For example, the cells can be collected using a cell picking system (AS ONE Corporation) or CellCelector (Automated Lab Solution GmbH).

**In** a case where two or more cells including the target cell are introduced into the positive microwell, for example, after the two or more cells are collected in a culture medium for cell culture, the two cells may be separated from each other, and any one of the two cells may be adopted as the target cell.

**In** addition, after collecting two cells in a buffer solution or the like, the nucleic acids encoding two types of objective substances can be isolated and acquired by the method described below, and any one of the nucleic acids can be employed as a target nucleic acid (target gene).

In addition, the microwell containing two or more cells may be excluded from the collection target as the well in which the target cell has not been identified.

(Manufacturing method of nucleic acid or manufacturing method of objective substance)

The manufacturing method of a nucleic acid according to the embodiment of the present invention includes acquiring a nucleic acid encoding an objective substance from the target cell obtained by the screening method.

In addition, the manufacturing method of an objective substance according to the embodiment of the present invention includes manufacturing an objective substance using the nucleic acid obtained by the manufacturing method of a nucleic acid according to the embodiment of the present invention.

The manufacture of the objective substance using the nucleic acid also preferably includes, for example, introducing the nucleic acid into a host cell to acquire a recombinant cell that expresses the objective substance, culturing the recombinant cell to acquire the objective substance from the culture product.

As a method of acquiring a nucleic acid encoding an objective substance from a target cell, a known method can be used. Examples thereof include a method of synthesizing cDNA by combining a reverse transcription reaction and a PCR method and isolating a target nucleic acid from the cDNA.

As a method of acquiring a recombinant cell that expresses an objective substance, a known method can be used. Examples thereof include a method of appropriately incorporating the acquired nucleic acid into a vector, and introducing the vector into a host cell such as Escherichia coli, yeast, or mammalian cells (for example, CHO cells, HEK293 cells, or NS0 cells) to acquire a target recombinant cell.

As a method of culturing the recombinant cell and acquiring an objective substance from the culture product, a known method can be used. For example, the recombinant cell can be cultured, and the objective substance can be acquired from the culture product (for example, the culture supernatant).

In a case where the objective substance is an antibody, the isolation of the antibody gene from the target cell can be performed, for example, by a combination of the methods described in WO2009/091048A, WO2009/110606A, and WO2011/027808A, or by the method (MAGrahd method) described in Nobuyuki Kurosawa, Megumi Yoshioka, Rika Fujimoto, Fuminori Yamagishi, and Masaharu Isobe, "Rapid production of antigen-specific monoclonal antibodies from a variety of animals", BMC Biology, 10:80, 2012.

A recombinant cell that expresses a complete antibody, a functional antibody fragment, a single-chain antibody, or a multispecific antibody may be constructed by modifying the isolated antibody gene. Similarly, a recombinant cell that expresses a complete human antibody, a humanized antibody, or a chimeric antibody may be constructed. Furthermore, a recombinant cell that expresses a felinized antibody or a caninized antibody may be constructed.

**In** a case where the target cell can be stably cultured, the target cell itself can be cultured to acquire an objective substance such as an antibody from the culture product.

That is, the manufacturing method of an objective substance is also preferably a method including culturing the target cell identified by the screening method according to the embodiment of the present invention and acquiring the objective substance from the culture product.

As a method of purifying an objective substance such as an antibody from the culture product, a known method can be used. For example, various chromatography such as affinity, ion exchange, and gel filtration can be employed. Examples of the ligand in affinity chromatography include protein A, protein G, an anti-FLAG antibody, an anti-V5 antibody, and the like.

### <Pharmaceutical composition>

A first aspect of a manufacturing method of a pharmaceutical composition according to the embodiment of the present invention includes containing, as an active ingredient, the nucleic acid manufactured by the manufacturing method of a nucleic acid according to the embodiment of the present invention in combination with a pharmaceutically acceptable carrier or additive.

A second aspect of a manufacturing method of a pharmaceutical composition according to the embodiment of the present invention includes containing, as an active ingredient, the objective substance manufactured by the manufacturing method of an objective substance according to the embodiment of the present invention in combination with a pharmaceutically acceptable carrier or additive

The objective substance manufactured by the manufacturing method of an objective substance according to the embodiment of the present invention, for example, an antibody, is useful as an active ingredient of a pharmaceutical composition (therapeutic agent).

The pharmaceutical composition can contain an objective substance such as an antibody manufactured by the manufacturing method of an objective substance according to the embodiment of the present invention, and a pharmaceutically acceptable carrier or additive.

Preferably, the pharmaceutical composition blocks or activates an intracellular information transmission mechanism that is specific to a cell membrane protein which is a target substance.

The pharmaceutical composition can be administered orally or parenterally throughout the body or locally. Examples of the form of administration include an injection type, a nasal administration type, a pulmonary administration type, and a transdermal administration type, and the like, and the form of administration is not particularly limited. **In** a case of an injection type, the administation can be performed throughout the body or locally, for example, by intravenous injection, intramuscular injection, intraperitoneal injection, subcutaneous injection, or the like. **In** addition, the method of administration can be appropriately selected depending on the age and symptoms of the patient.

**In** a case where the objective substance is an antibody, the dose of the antibody is not particularly limited, but for example, it can be selected in a range of 0.0001 mg to 1000 mg per kg of body weight per administration. Alternatively, the dose can be selected, for example, in a range of 0.001 to 100,000 mg/body per patient, without particular limitation.

The pharmaceutical composition can be formulated according to a conventional method (for example, Remington's Pharmaceutical Science, latest edition, Mark Publishing Company, Easton, U.S.A). Examples of the carrier or the additive include surfactants (PEG, Tween, and the like), excipients, antioxidants (ascorbic acid and the like), colorants, fragrances, preservatives, stabilizers, buffering agents (phosphoric acid, citric acid, other organic acids, and the like), chelating agents (EDTA and the like), suspending agents, isotonic agents, binding agents, disintegrating agents, lubricants, fluidity promoters, taste modifiers, and the like. Specific examples thereof include light anhydrous silicic acid, lactose, crystalline cellulose, mannitol, starch, carmellose calcium, carmellose sodium, hydroxypropyl cellulose, hydroxypropyl methyl cellulose, polyvinyl acetal diethylamino acetate, polyvinylpyrrolidone, gelatin, medium-chain fatty acid triglyceride, polyoxyethylene hydrogenated castor oil 60, sucrose, carboxymethyl cellulose, corn starch, inorganic salts, and the like. **In** addition, other low-molecular-weight polypeptides; proteins such as serum albumin, gelatin, and immunoglobulin; and amino acids such as glycine, glutamine, asparagine, arginine, and lysine may be contained.

In a case where the pharmaceutical composition is prepared as an aqueous solution for injection, examples thereof include saline, an isotonic solution containing glucose or other auxiliary agents, for example, D-sorbitol, D-mannose, D-mannitol, or sodium chloride, and an appropriate solubilizing agent, for example, alcohol (ethanol or the like), polyalcohol (propylene glycol, PEG, or the like), or a nonionic surfactant (Polysorbate 80, HCO-50), or the like, may be used in combination.

In addition, as necessary, the antibody as an active component can be enclosed in microcapsules (microcapsules such as hydroxymethyl cellulose, gelatin, and poly[methyl methacrylate]) or can be made into a colloidal drug delivery system (liposomes, albumin microspheres, microemulsions, nanoparticles, nanocapsules, and the like) (see "Remington's Pharmaceutical Science 16th edition", Oslo Ed. (1980), and the like).

Furthermore, a technique of directly fusing an antibody with another drug to enhance a therapeutic effect is known, and this technique can be applied to the pharmaceutical composition.

In addition, it is also conceivable to incorporate the nucleic acid (gene) obtained in the present invention, for example, the antibody gene, into a gene therapy vector to obtain a gene therapy drug. Examples of the method of administering the gene therapy agent (recombinant vector) include a method of directly administering a naked plasmid, a method of administering a naked plasmid by packaging it in liposomes or the like, a method of administering a naked plasmid by incorporating it into various viral vectors such as a retrovirus vector, an adenovirus vector, a vaccinia virus vector, a poxvirus vector, an adeno-associated virus vector, and an HVJ vector (see Adolph "Viral Genome Method", CRC Press, Florida (1996)), a method of administering a naked plasmid by coating it on a bead carrier such as colloidal gold particles (WO1993/017706A), and the like.

That is, the gene therapy drug may be administered by any method as long as the antibody as an active ingredient is expressed in vivo and the action thereof can be exhibited. Preferably, a sufficient amount is administered by an appropriate non-oral route. Examples of the non-oral route include a method via a mucous membrane route such as injection, infusion, or gas-induced particle impact method (by an electron gun or the like) via a route through a vein, an abdominal cavity, a subcutaneous tissue, an intradermal tissue, a fat tissue, a mammary gland tissue, inhalation, or a muscle, collunarium, and the like. Furthermore, the gene therapy drug may be administered to cells by ex vivo liposome transfection, particle bombardment (US4945050A), or virus infection, and the cells may be reintroduced into an animal for administration.

### (Reagent for detecting target substance)

The reagent for detecting a target substance according to the embodiment of the present invention includes a reagent for detecting a desired target substance, which contains the objective substance manufactured by the above-described method. For example, the antibody (objective substance) is brought into contact with a blood cell derived from a human or a non-human mammal using a reagent containing the antibody manufactured by the manufacturing method of an objective substance according to the embodiment of the present invention. Furthermore, a labeling substance consisting of a fluorescent substance or a coloring agent is brought into direct or indirect contact with the cell. Then, the expression of a desired target substance (for example, a cell membrane protein) can be detected by flow cytometry or a plate reader. **In** addition, the antibody can be brought into contact with a pathological tissue section derived from a human or a non-human mammal using the reagent to detect the expression of a desired target substance.

Furthermore, a target substance detection kit including the reagent can be constructed. For example, the reagent can be combined with a labeling substance or the like to prepare a target substance detection kit.

The present invention includes a method of detecting a target substance, using the objective substance manufactured by the above-described method. The present invention includes the use of the objective substance manufactured by the above-described method for the detection of the target substance.

### Examples

Hereinafter, the present invention will be described in detail using examples. Materials, using amounts, proportions, treatment details, treatment content, and the like shown in the following examples can be appropriately changed without departing from the gist of the present invention. Therefore, the scope of the present invention is not limited to the specific examples described below.

### (Example 1: preparation of target protein-displaying EV)

### <Experiment method>

293T cells in which EphA2 (single transmembrane), 5-hydroxytryptamine receptor 1A (7 pass transmembrane, HA tag fused to the N-terminal), or Claudin-5 (4 pass transmembrane), which are transmembrane proteins, was transiently and forcibly expressed by a lipofectamine method with Lipofectamine 2000 Transfection Reagent (Invitrogen 11668027) were cultured in a serum-free medium for 1 to 3 days, and the EVs were purified using MagCapture (registered trademark) Exosome Isolation Kit PS Version 2 (manufactured by FUJIFILM Wako Pure Chemical Corporation, 290-84103) from the culture supernatant. The purified EV was subjected to particle number measurement with NanoSight (a nanoparticle analysis system, manufactured by Quantum Design Japan, Inc.). EV derived from cells (TF(-)) treated with only a transfection reagent was also prepared as a negative control for each cell, and the particle size distribution thereof was analyzed.

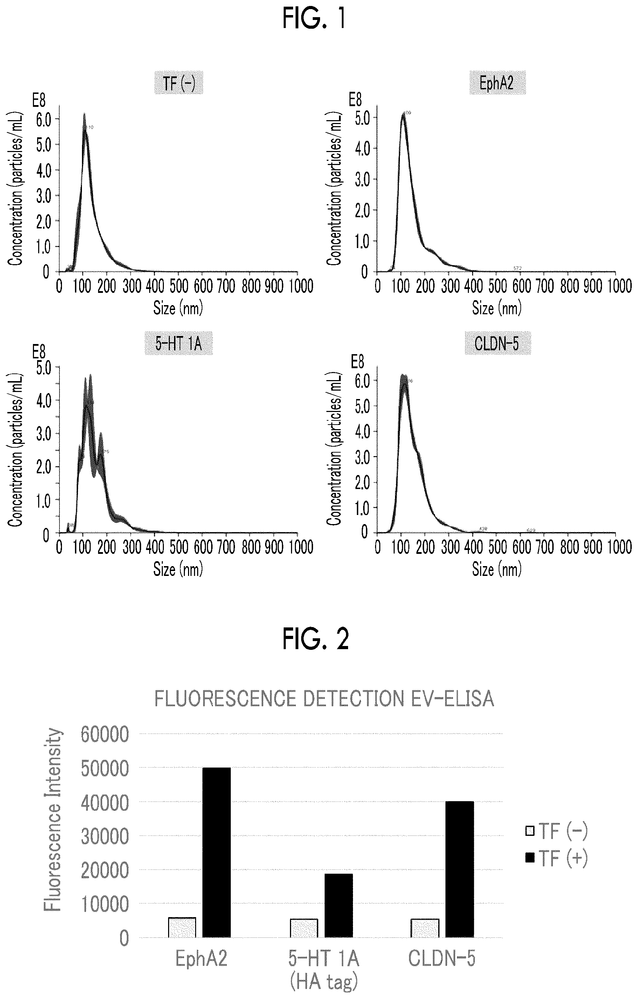

The analysis results are shown in Fig. 1.

### <Result>

Fig. 1 is a graph showing an analysis result of a particle size distribution plotted with a horizontal axis as a particle diameter and a vertical axis as a particle number concentration.

From Fig. 1, it was confirmed that particles (EV) of about 100 to 300 nm were collected from both the negative control and the cell supernatant in which each transmembrane protein was expressed, by the purification of EV from the culture supernatant and the analysis by NanoSight.

### (Example 2: confirmation of target protein expressing EV)

### <Experiment method>

The fluorescence detection EV-ELISA was performed by the following method using the EV derived from the target protein-expressing cell, which was prepared in Example 1. EV derived from cells (TF(-)) treated only with a transfection reagent was also prepared and analyzed as a negative control.

· EV-ELISA: 1.5 µg/mL of Mouse TIM4/Human Fc Chimera, recombinant (FUJIFILM Wako Pure Chemical Corporation, 137-18511) was coated on a 96 well plate at 4°C overnight. After blocking with Block Ace (manufactured by KAC Co., Ltd.) for 1 hour on the next day, reaction with each of the EV particles (2.5 × 10⁸ particles/well) obtained in Example 1 was performed at room temperature for 2 hours. Next, 1 µg/mL various antibodies (EphA2: BioLegend 356802, HA: SIGMA H3663, Claudin-5: FUJIFILM Wako Pure Chemical Corporation, 014-28101) were added thereto, the mixture was allowed to react at room temperature for 1 hour, and then 10 µg/mL a biotin-labeled secondary antibody (FUJIFILM Wako Pure Chemical Corporation, 512-20531) was added thereto and the mixture was allowed to react at room temperature for 1 hour. Finally, 100 µL of 2 µg/mL streptavidin-Alexa Fluor (registered trademark) 488 (FUJIFILM Wako Pure Chemical Corporation, 560-77841) was added thereto, and the mixture was allowed to react at room temperature for 1 hour. Thereafter, the fluorescence intensity was measured with a plate reader. The measurement results are shown in Fig. 2.

### <Result>

Fig. 2 is a graph showing values of fluorescence intensity measured in each transmembrane protein expressing EV and a negative control.

From the results shown in Fig. 2, in the results of EV-ELISA by fluorescence detection, it was confirmed that the signal of the target protein could be detected from each EV, and the target protein was presented on the EV. In addition, it was confirmed that the S/B ratio was about 2.5 to 8.5 times as compared with the fluorescence signal of the target protein non-expressing EV (cell-derived EV without gene transfer).

### (Example 3: verification in microchamber of ASONE Cell Picking System)

### <Experiment method>

A chamber in which about 200,000 wells having a diameter of 20 µm were present was coated with 20 µg/mL of Tim4 at 4°C overnight. After blocking with BlockAce for 1 hour on the next day, reaction with 100 × 10⁹ particles/mL of EV particles in which EphA2 was expressed and obtained in Example 1 described above was performed at room temperature for 2 hours. Next, 3.5 × 10⁵ cells/mL of spleen cells derived from a rabbit immunized with EphA2 and 10 µg/mL of a secondary antibody (Biotin-labeled) (FUJIFILM Wako Pure Chemical Corporation, 564-72501) were added thereto, and reacted at 37°C and a CO₂ concentration of 5% for 1 hour, and finally 2 µg/mL of Abzine-labeled Alexa Fluor (registered trademark) 488 (manufactured by FUJIFILM Wako Pure Chemical Corporation) was added thereto, and the mixture was allowed to react at room temperature for 1 hour, and then imaging was performed using an ASONE Cell Picking System. The splenic cells were acquired by DNA immunization by administering the antigen expression plasmid to a rabbit.

### <Result>

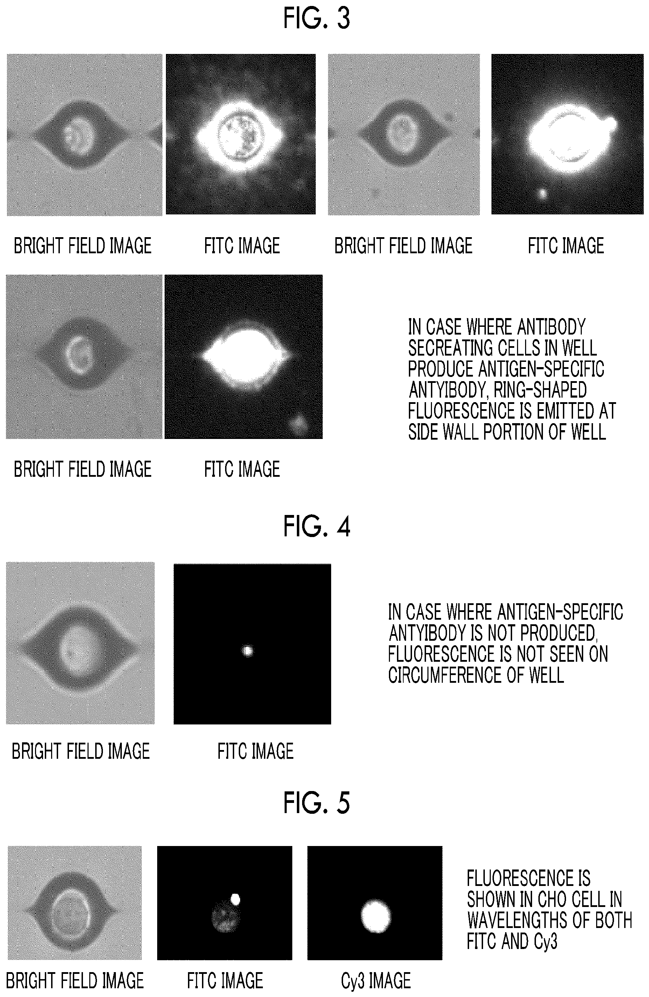

Fig. 3 shows an example of a result of imaging performed by an ASONE Cell Picking System. From Fig. 3, in the well in which the cell (positive cell) producing an antibody that specifically binds to EphA2 on EV was present, the vicinity of the side wall portion of the well was detected by fluorescence in a ring shape. In a case where 200,000 chambers were scanned, 8 positive cells were confirmed. As a reference, Fig. 4 shows an example of a result of imaging a cell (negative cell) that does not produce an antibody that specifically binds to EphA2 on EV. In the type of B cell that is easy to hold an antibody on a membrane, the labeled secondary antibody is bound to the antibody on the membrane. Therefore, the B cell itself is detected by fluorescence as shown in Fig. 4, but no ring-shaped fluorescence detection is observed in the vicinity of the side wall.

### (Example 4: comparison between EV-ELISA and Cell ELISA in ASONE Cell Picking System)

### <Experiment method>

The method (Cell-ELISA) described in WO2020/171020A was performed, and a comparison with EV-ELISA was performed. As the analysis cell in the Cell-ELISA (first cell in WO2020/171020A), a stable cell line of CHO-K1 expressing EphA2 was used, and as the second cell in WO2020/171020A, an Epha2-immunized rabbit spleen cell used in Example 3 was used. The analysis conditions were as follows.

5 × 10⁵ cells/mL of EphA2 stable expression CHO-K1 cells were seeded in a chamber having about 85,000 wells having a diameter of 30 µm, and stained with a 10 µmol/L CytoRed solution (FUJIFILM Wako Pure Chemical Corporation, 342-08531). Next, 5 × 10⁵ cells/mL of spleen cells derived from Epha2-immunized rabbits and 10 µg/mL of a secondary antibody (FITC-labeled) (FUJIFILM Wako Pure Chemical Corporation, 563-77951) used in Example 3 were added thereto, and the mixture was allowed to react at 37°C and a CO₂ concentration of 5% for 1 hour, and then imaging was performed using an ASONE Cell Picking System.

### <Result>

Fig. 5 shows an example of the imaging result of the positive cells observed by Cell-ELISA. From Fig. 5, the CHO cells have fluorescence in the Cy3 image, and both the small B cells and the large CHO cells have fluorescence in the FITC image. That is, it can be seen that in the positive cells, the CHO cells are recognized to have fluorescence in both the Cy3 image and the FITC image.

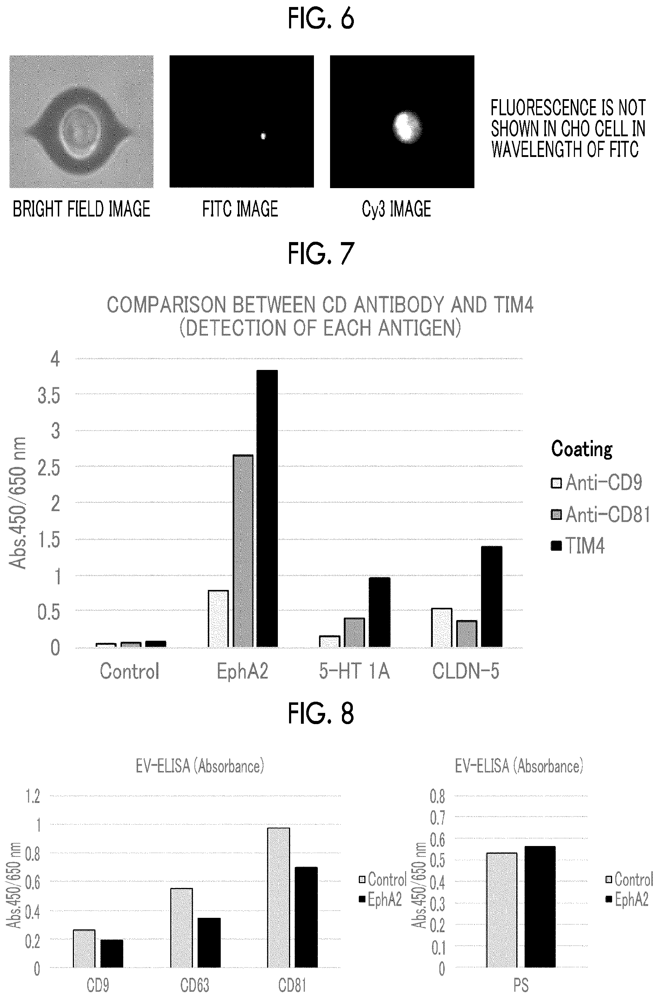

Fig. 6 shows an example of the imaging result of the negative cells observed by Cell-ELISA. From Fig. 6, the CHO cells have fluorescence in the Cy3 image, and only the small B cells have fluorescence in the FITC image. That is, it can be seen that in the negative cells, the CHO cells are recognized to have fluorescence in the Cy3 image, but are not recognized to have fluorescence in the FITC image.

Table 1 shows the results of comparing EV-ELISA with Cell ELISA.

From Table 1, the number of wells that can be analyzed in a case of using Cell-ELISA was 43,079 wells, whereas the number of wells in a case of using EV-ELISA was 140,416 wells as shown in Example 3 described above, which was an increase of about 3 times. Here, the count of the number of wells that can be analyzed is determined as follows. In the Cell-ELISA, the wells are filled with CHO cells (96%: 82,682 wells) and filled with spleen cells (50%: 43,079 wells). **In** the EV-ELISA, the number of wells in which the spleen cells are filled is counted as the number of wells that can be analyzed (71%: 140,416 wells). **In** addition, the number of positive cells was 46 in the EV-ELISA with respect to 3 in the Cell-ELISA. Furthermore, in a case where the positive rate with respect to the number of wells that can be analyzed was determined, the positive rate was 0.007% in Cell-ELISA, whereas the positive rate was 0.03% in EV-ELISA, and the detection sensitivity was about 4 times higher than that of the related art. **In** addition, in the Cell-ELISA, the standard for determining whether the obtained merged image is positive or negative is ambiguous, and it is difficult to visually determine how much fluorescence is detected in CHO cells to be positive. However, in the EV-ELISA, the determination can be made by confirming the fluorescence at the position of the side wall of the well (specifically, the ring-shaped fluorescence is confirmed). Therefore, the visual determination is easy, and the screening sensitivity is significantly improved.

**In** addition, in the Cell-ELISA, the number of wells (that is, the filling rate) in which CHO cells are filled in the wells and spleen cells are filled in the wells was examined to be increased. The seeding conditions of the spleen cells were examined to be 2.5 × 10⁵ cells, 5 × 10⁵ cells, and 10 × 10⁵ cells, but the filling rate did not increase by 50% or more. That is, the proportion of the number of wells that can be analyzed was about 50% of the total number of wells. On the other hand, in the EV-ELISA, since the well in which the spleen cells are filled in the well is the wells that can be analyzed as it is, the proportion of the number of the wells that can be analyzed was about 71% with respect to the total number of wells as described above. From the above, it can be seen that the EV-ELISA has a higher proportion of the wells that can be analyzed as compared with the Cell-ELISA, and a larger number of wells can be used for the analysis.

Furthermore, from the viewpoint of screening efficiency, in a case where the positive rate with respect to the number of wells was determined, the positive rate was 0.0035% in Cell-ELISA, whereas the positive rate was 0.023% in EV-ELISA, and the screening efficiency was improved by about 6 times as compared with the method of the related art.

**[Table 1]**

| Method | Number of wells [wells] | Number of wells that can be analyzed [wells] | Number of positive cells [wells] | Positive rate with respire to number of analyses [%] | Positive rate with respire to number of wells [%] |
|---|---|---|---|---|---|
| Cell-ELISA | 84,640 | 43,079 | 3 | 0.007 | 0.0035 |
| EV-ELISA | 196,000 | 140,416 | 46 | 0.03 | 0.023 |

### (Example 5: superiority of EV solid-phasing in PS (phosphatidylserine)-TIM4 affinity reaction)

### <Experiment method>

96 well plate was coated with each of 1.5 µg/mL of Tim4, 1 µg/mL of Anti-CD9 antibody (FUJIFILM Wako Pure Chemical Corporation, 013-28171), or 1 µg/mL of Anti-CD81 antibody (FUJIFILM Wako Pure Chemical Corporation, 010-28223) at 4°C overnight. After blocking with Block Ace (manufactured by KAC Co., Ltd.) for 1 hour on the next day, reaction with each of the EV particles (2.5 × 10⁸ particles/well) obtained in Example 1 was performed at room temperature for 2 hours. Next, 1 µg/mL of various antibodies (EphA2: BioLegend 356802, HA: SIGMA H3663, Claudin-5: FUJIFILM Wako Pure Chemical Corporation, 014-28101) were added thereto, and the mixture was allowed to react at room temperature for 1 hour, and then reacted with an HRP-labeled secondary antibody (FUJIFILM Wako Pure Chemical Corporation, 512-20531 and 567-80161) at room temperature for 1 hour. Finally, 100 µL of a TMB solution (FUJIFILM Wako Pure Chemical Corporation, 208-17371) was added thereto, the mixture was allowed to react at room temperature for 15 minutes, and then the reaction was stopped with 1 M HCl. The absorbance A at 450 nm and the absorbance B at 650 nm were measured for each well with a plate reader, and the absorbance A/B (Abs. 450/650 nm) was calculated. The measurement results are shown in Fig. 7. In addition, the TF(-)EV obtained in Example 1 described above was used as a negative control.

### <Result>

From the results shown in Fig. 7, it can be seen that the absorbance measurement value is the highest in a case where Tim4 is used, as compared with a case where the Anti-CD9 antibody or the Anti-CD81 antibody is used for the EV solid-phasing. That is, it is considered that the detection sensitivity is most excellent in a case where Tim4 is used for the immobilization of EV.

### (Example 6: change in amount of CD marker protein and PS on EV)

### <Experiment method>

96 well plates were coated with 1.5 µg/mL of Tim4 at 4°C overnight. After blocking with Block Ace (manufactured by KAC Co., Ltd.) for 1 hour on the next day, reaction with EphA2 expressing EV particles (0.5 × 10⁸ particles/well) obtained in Example 1 was performed at room temperature for 2 hours. 1 µg/mL of various CD marker protein antibodies (FUJIFILM Wako Pure Chemical Corporation, CD9: 0.013-28171, CD63: 0.012-27063, and CD81: 0.010-28223) or Annexin V Biotin (EXBIO EXB0031) was added thereto, the mixture was allowed to react at room temperature for 1 hour, and then reacted with 1 µg/mL of HRP-labeled secondary antibody (FUJIFILM Wako Pure Chemical Corporation, 512-20531 or 567-80161) or Poly-HRP Streptavidin (Thermo N200) at room temperature for 1 hour. Finally, 100 µL of a TMB solution (FUJIFILM Wako Pure Chemical Corporation, 208-17371) was added thereto, the mixture was allowed to react at room temperature for 15 minutes, and then the reaction was stopped with 100 µL of 1 M (mol/L) HCl. The absorbance A at 450 nm and the absorbance B at 650 nm were measured for each well with a plate reader, and the absorbance A/B was calculated. The measurement results are shown in Fig. 8. In addition, the target protein non-expressing EV (cell-derived EV without gene transfer) obtained in Example 1 described above was used as a control.

### <Result>

Fig. 8 is a graph showing absorbance measurement values measured in EphA2 protein expressing EV and control.

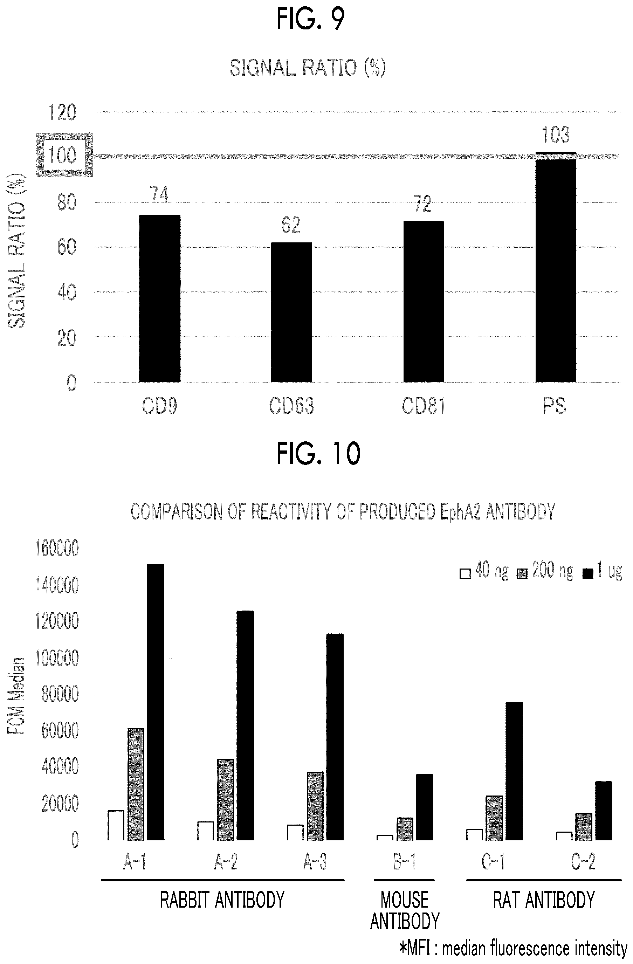

Fig. 9 is a graph showing a value of a signal/background ratio (S/B ratio) obtained by dividing absorbance measurement value measured in the EphA2 protein expression EV by an absorbance measurement value measured in the control.

From the results shown in Fig. 8, it can be seen that the expression level of the CD marker protein on the EV decreases by gene transfer, but the amount of PS does not decrease.

In addition, from the results shown in Fig. 9, it was confirmed that the S/B ratio with respect to the CD marker protein was reduced by about 20% to 40% as compared with the absorbance measurement value of the target protein non-expressing EV (cell-derived EV without gene transfer).

This is considered to be because the expression levels of CD marker proteins such as CD9 and CD81 decrease by gene transfer, but the amount of PS does not decrease or is not easily to decrease.

### (Example 7: confirmation of activity of acquired antibody)

### <Experiment method>

The antigen binding properties of the rabbit antibodies (A-1, A-2, and A-3) derived from B cells, which were acquired by the screening method according to Example 3, were confirmed by flow cytometry analysis. For the analysis, EphA2 expressing cells produced by the method shown in Example 1 were used, and non-expressing cells were also produced to confirm the specific binding to the antigen, and the analysis was performed at the same time. In addition, as a comparative target, a mouse anti-EphA2 antibody (B-1) and rat antibodies (C-1, C-2) produced by a hybridoma method were also analyzed at the same time. The spleen cells used for the production of the hybridoma were acquired by DNA immunization by administering an antigen expression plasmid to a mouse or a rat.

### Specifically, the following procedures were performed.

The culture medium was discarded, and the cells were washed with PBS (-) for the EphA2 expressing cells and the non-expressing cells. After washing, Cell Dissociation Buffer (6-well plate: 500 µL, 150 mm dish: 3 mL) was added and incubated at 37°C for about 5 minutes. After incubation, 3% FBS/PBS (6-well plate: 1 mL, 150 mm dish: 12 mL) was added, the cells were suspended with a pipette, and the suspension was collected in a centrifuge tube. Centrifugation was performed at 1,200 rpm for 3 minutes, the cells were allowed to stand to be pellet down, and then 3% FBS/PBS (6-well plate: 1200 µL, 150 mm dish: 10 mL) was added to the cells to resuspend the cells. The cell suspension was added to a 96 well plate (round bottom, Corning 3788) at 200 µL (approximately 2 × 10⁵ cells) per well, and then centrifuged at 400 x g for 5 minutes.

The antibody was diluted with FCM Buffer (3% FBS/PBS) such that the antibody concentration was 1 µg/mL, 200 ng/mL, or 40 ng/mL, and a primary antibody solution was prepared. The supernatant of the cell culture was removed, the culture supernatant stock solution at 50 µL per well and the primary antibody solution (diluted with 3% FBS/PBS) at 25 µL per well were added, and the mixture was suspended and incubated at 4°C for 30 minutes. After incubation, 3% FBS/PBS was added thereto at 150 µL per well of a 96-well plate thereto, and centrifugation was carried out at 4°C and 400 xg for 5 minutes. The supernatant was removed, and a PE-labeled secondary antibody solution (Southern Biotech, 1030-09 or 4030-09) (diluted with 3% FBS/PBS such that the antibody concentration was 1 µg/mL) was added at 25 µL per well, and the cells were suspended and incubated at 4°C for 30 minutes. After incubation, 3% FBS/PBS was added thereto at 150 µL per well of a 96-well plate thereto, and centrifugation was carried out at 4°C and 400 xg for 5 minutes. The supernatant was removed, and 3% FBS/PBS was added at 200 µL per well. The fluorescence intensity of the fluorescently labeled cells was measured with a flow cytometer (CytoFLEX, manufactured by Beckman Coulter, Inc.). The measurement results are shown in Fig. 10.

### <Result>

Fig. 10 is a graph obtained by calculating the median value of the FCM shift intensity, subtracting the median value of the non-expressing cells from the median value of the expressing cells. It was confirmed that all of the acquired rabbit antibodies specifically reacted with the EphA2 expressing cell, and an antigen-specific antibody was acquired by the screening method according to the embodiment of the present invention. **In** addition, the binding force of the rabbit antibody acquired by the screening method according to the embodiment of the present invention was stronger than that of the mouse antibody and the rat antibody produced by the hybridoma method. According to the screening method according to the embodiment of the present invention, it is suggested that an antibody having a high binding force to an antigen can be acquired.

## Claims

1. A screening method for a target cell which is a cell that produces an objective substance, the screening method comprising the following (1) to (3):
(1) immobilizing a vesicle having a target substance on a membrane surface in a microwell;
(2) introducing a candidate cell obtained from a cell population containing the target cell and a labeled substance which is a substance that binds to the objective substance into the microwell in which the vesicle has been immobilized; and
(3) identifying the target cell by detection of a label of the objective substance bound to the target substance.

2. The screening method according to claim 1,
wherein the target substance is a membrane protein.

3. The screening method according to claim 1,
wherein the objective substance is an antibody.

4. The screening method according to claim 1,
wherein the target cell is a B cell derived from a spleen cell or lymphoid tissue.

5. The screening method according to claim 1,
wherein the target cell is a CHO cell or a hybridoma.

6. The screening method according to claim 1,
wherein in the (1), the vesicle is immobilized in the microwell using at least one selected from the group consisting of a Tim family protein, annexin V, an anti-CD9 antibody, an anti-CD63 antibody, and an anti-CD81 antibody.

7. The screening method according to claim 6,
wherein in the (1), the vesicle is immobilized in the microwell using the Tim family protein.

8. The screening method according to claim 1,
wherein in the (2), the candidate cell to be introduced into the microwell is one cell that has been isolated.

9. The screening method according to claim 1,
wherein the vesicle is an extracellular vesicle.

10. The screening method according to claim 1,
wherein the vesicle has a diameter of 30 to 1,000 nm.

11. The screening method according to claim 1,
wherein the detection in the (3) is fluorescence detection.

12. The screening method according to claim 11,
wherein the identifying of the target cell by the fluorescence detection is performed by excitation at one wavelength and fluorescence measurement at one wavelength.

13. The screening method according to claim 11,
wherein in the (3), the target cell is identified by detecting a well in which fluorescence is confirmed at a position of a side wall of the microwell.

14. The screening method according to claim 1, further comprising the following (4):
(4) collecting the target cell identified in the (3).

15. A manufacturing method for a nucleic acid comprising:
acquiring a nucleic acid encoding an objective substance from the target cell obtained by the screening method according to any one of claims 1 to 14.

16. A manufacturing method for an objective substance, comprising:
manufacturing an objective substance using the nucleic acid obtained by the manufacturing method for a nucleic acid according to claim 15.
